(19) **Europäisches Patentamt / European Patent Office / Office européen des brevets**

(11) **EP 3 797 096 B1**

(12) **FASCICULE DE BREVET EUROPEEN**

(45) Date de publication et mention de la délivrance du brevet:
**31.12.2025 Bulletin 2026/01**

(21) Numéro de dépôt: **19725204.2**

(22) Date de dépôt: **23.05.2019**

(51) Classification Internationale des Brevets (IPC):
**C07C 7/04** (2006.01)     **C07C 6/04** (2006.01)
**B01D 3/42** (2006.01)     **C07C 11/09** (2006.01)
**C07C 11/08** (2006.01)     **C07C 11/06** (2006.01)
**B01D 3/14** (2006.01)

(52) Classification Coopérative des Brevets (CPC):
(C-Sets disponibles)
**C07C 6/04; B01D 3/143; B01D 3/4261; C07C 7/04**
(Cont.)

(86) Numéro de dépôt international:
**PCT/EP2019/063387**

(87) Numéro de publication internationale:
**WO 2019/224330 (28.11.2019 Gazette 2019/48)**

(54) **PROCÉDÉ DE PRODUCTION D'UN FLUX DE PROPYLÈNE ET INSTALLATION ASSOCIÉE**

VERFAHREN ZUR HERSTELLUNG EINES PROPYLENSTROMS UND ZUGEHÖRIGE ANLAGE

METHOD FOR PRODUCING A STREAM OF PROPYLENE AND ASSOCIATED FACILITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.05.2018 FR 1854284**

(43) Date de publication de la demande:
**31.03.2021 Bulletin 2021/13**

(73) Titulaire: **Technip Energies France**
**92741 Nanterre Cedex (FR)**

(72) Inventeurs:
• **REICH, Véronique**
**92420 Vaucresson (FR)**
• **DESTOUR, Bruno**
**92500 Rueil Malmaison (FR)**

(74) Mandataire: **Withers & Rogers**
**Withers & Rogers LLP**
**2 London Bridge**
**London SE1 9RA (GB)**

(56) Documents cités:
**WO-A1-2005/110951     WO-A1-2017/003818**

**(Cont. page suivante)**

(52) Classification Coopérative des Brevets (CPC):
(Cont.)

C-Sets
**C07C 6/04, C07C 11/06;**
**C07C 7/04, C07C 11/08;**
**C07C 7/04, C07C 11/09**

# EP 3 797 096 B1

**Description**

La présente invention concerne un procédé de production d'un flux de propylène, selon le préambule de la revendication 1.

WO2017/003818 décrit un procédé de métathèse qui sépare les composés C4 paraffiniques dans une colonne située en aval du depropyléniseur.

Un tel procédé mis en œuvre notamment au sein ou en parallèle d'une unité de craquage d'hydrocarbures, notamment d'une unité de vapocraquage et/ou d'une raffinerie (unité de craquage catalytique FCC, ou autres).

Le propylène est produit selon le procédé par mise en contact d'un flux de 2-butène et d'un flux d'éthylène dans un réacteur de métathèse.

Dans un procédé connu, le flux de 2-butène et le flux d'éthylène pour la métathèse proviennent d'une unité de vapocraquage. Une coupe C4 de raffinerie (craquage catalytique ou autres) est éventuellement utilisée comme une autre source de butène. La (ou les) coupe(s) C4 sont usuellement traitées en amont de la métathèse pour obtenir une charge exempte de butadiène, pauvre en isobutène et avec un ratio 2-butène/1-butène orienté le plus possible vers le 2-butène. Le 2-butène peut également être obtenu par dimérisation d'éthylène en 1-butène, puis par isomérisation du 1-butène en 2-butène.

Dans le cas d'une coupe C4 de vapocraquage, celle-ci est typiquement traitée dans une unité d'hydrogénation sélective du butadiène, assurant également l'hydroisomérisation du 1-butène en 2-butène, avant d'être traitée dans une colonne de distillation afin d'éliminer l'isobutène. Alternativement, le butadiène est extrait grâce à une unité dédiée et/ou l'isobutène est extrait via une unité de type MTBE. Le raffinat extrait de ce(s) unité(s), riche en 2-butène est alors envoyé vers le réacteur de métathèse.

Une section de séparation est par ailleurs prévue en aval de l'unité de métathèse pour traiter l'effluent. Une première colonne de distillation (dénommée ci-après « Dééthyléniseur ») assure la séparation de l'éthylène n'ayant pas réagi de la coupe C3+ produite par le réacteur de métathèse.

L'éthylène récupéré en tête est séparé en une purge d'éthylène et en un recyclage d'éthylène au réacteur de métathèse.

La coupe C3+ produite en fond du Dééthyléniseur est envoyée dans une deuxième colonne de distillation (dénommée ci-après « Dépropyléniseur »). Le propylène est produit en tête de cette colonne. Une coupe riche en hydrocarbures en C4 est soutirée en phase liquide entre le plateau d'alimentation et le fond de la colonne, puis est recyclée vers le réacteur de métathèse.

Un tel procédé n'est pas optimal en termes de rendement ultime en propylène et/ou de durée de cycle des réacteurs.

Tout d'abord, le recyclage vers le réacteur des composés paraffiniques (isobutane, n-butane, ...) est inutile, puisque ces composés saturés sont par nature inertes vis-à-vis de la réaction de métathèse.

Par ailleurs, la réaction de métathèse de l'isobutène avec l'éthylène ne donne pas de nouveaux produits, tandis que la réaction de métathèse de l'isobutène avec lui-même et/ou les autres hydrocarbures oléfiniques en C4 est limitée en présence d'excès d'éthylène. Il n'est donc pas avantageux de recycler l'isobutène vers le réacteur de métathèse.

Lorsque ces composés sont présents dans l'unité en quantité importante, ils bloquent les sites actifs du catalyseur pour peu, voire pas de rendement, et ils s'accumulent dans le recycle riche en hydrocarbures en C4. Ceci conduit à une surconsommation inutile et/ou à un une limitation de l'unité, sauf à devoir prévoir des équipements de capacité plus importante.

Par ailleurs, la coupe riche en hydrocarbures en C4 destinée à être recyclée vers le réacteur de métathèse est extraite du Dépropyléniseur par un soutirage en phase liquide dans la zone de fond de la colonne. Cet arrangement induit qu'une partie des composants lourds (notamment hydrocarbures en C6+) formés lors de la réaction de métathèse et/ou produits par des réactions secondaires sont également recyclés vers le réacteur. De tels composés sont source d'encrassement et de cokage prématuré du catalyseur, ce qui peut réduire la durée de cycle des catalyseurs utilisés pour la réaction de métathèse, et à terme leur durée de vie.

Un but de l'invention est donc d'obtenir un procédé de production de propylène de très haute qualité à partir d'un réacteur de métathèse, présentant un rendement amélioré et/ou une capacité augmentée, sans affecter significativement la structure des équipements utilisés pour mettre en œuvre le procédé.

A cet effet, l'invention a pour objet un procédé selon la revendication 1.

Le procédé selon l'invention peut comprendre l'une ou plusieurs des caractéristiques des revendications 2 à 8, prises isolément ou suivant toute combinaison techniquement possible.

L'invention a également pour objet un procédé de production d'un flux de propylène, comportant les étapes suivantes :

- introduction d'une coupe d'alimentation riche en hydrocarbures en C4 ou/et en C5, et d'au moins une coupe riche en éthylène dans un réacteur de métathèse;

3

- récupération d'un produit de métathèse à la sortie du réacteur de métathèse ;
- introduction du produit de métathèse dans un Dééthylèniseur ;
- production en tête du Dééthylèniseur d'un flux de tête riche en éthylène et en fond du Dééthylèniseur d'un flux d'alimentation;
- introduction du flux d'alimentation dans un Dépropylèniseur et récupération en fond du Dépropylèniseur d'un flux de fond contenant des hydrocarbures en C4+ ;
- récupération, à partir d'un flux de tête du Dépropylèniseur, du flux de propylène;
- prélèvement latéral d'un flux de recyclage riche en hydrocarbures en C4 ou/et en C5 et renvoi du flux de recyclage vers le réacteur de métathèse;

caractérisé en ce que le prélèvement latéral du flux de recyclage est effectué en phase gaz.

Le procédé selon l'invention ne comprend pas nécessairement de soutirage latéral, dans le Dépropylèniseur, d'une purge riche en hydrocarbures paraffiniques en C4 et/ou riche en isobutène. Il peut comprendre l'une ou plusieurs des caractéristiques mentionnées ci-dessus, prises isolément ou suivant toute combinaison techniquement possible.

L'invention a également pour objet une installation de production de propylène, selon la revendication 9.

L'installation selon l'invention peut comprendre l'une ou plusieurs des caractéristiques des revendications 10 à 15 ou la caractéristique suivante, prises isolément ou suivant toute combinaison techniquement possible :

- l'installation comporte un condenseur propre à au moins partiellement condenser le flux de tête issu du Dééthylèniseur pour former une fraction liquide et une purge vapeur, et un séparateur propre à fractionner la fraction liquide en un reflux introduit dans le Dééthylèniseur et en un flux de recyclage d'éthylène renvoyé vers le réacteur de métathèse.

L'invention a également pour objet une installation de production de propylène, comportant :

- un réacteur de métathèse alimenté par au moins une coupe d'alimentation riche en hydrocarbures en C4 ou/et en C5, et par au moins une coupe riche en éthylène, le réacteur de métathèse produisant un produit de métathèse;
- un Dééthylèniseur, alimenté par le produit de métathèse issu du réacteur de métathèse, le Dééthylèniseur produisant, en tête, un flux de tête riche en éthylène et produisant, en fond, un flux d'alimentation ;
- un Dépropylèniseur recevant le flux d'alimentation et produisant, en fond, un flux de fond contenant des hydrocarbures en C4+ et, en tête, un flux de tête riche en propylène ;
- un ensemble de récupération, à partir du flux de tête du Dépropylèniseur, d'un flux de propylène;
- un ensemble de prélèvement latéral d'un flux de recyclage riche en hydrocarbures en C4 ou/et en C5 et de renvoi du flux de recyclage vers le réacteur de métathèse

caractérisée en ce que l'ensemble de prélèvement latéral est propre à prélever un flux de recyclage en phase gaz.

L'installation selon l'invention comprend nécessairement un ensemble de soutirage latéral, dans le Dépropylèniseur, d'une purge riche en hydrocarbures paraffiniques en C4 et/ou riche en isobutène.

Elle peut comprendre l'une ou plusieurs des caractéristiques mentionnées ci-dessus, prises isolément ou suivant toute combinaison techniquement possible.

L'invention sera mieux comprise à la lecture de la description qui va suivre, donnée uniquement à titre d'exemple, et faite en se référant aux dessins annexés, sur lesquels :

- la figure 1 est un schéma synoptique fonctionnel d'une première installation de production destinée à la mise en œuvre d'un procédé selon l'invention ;
- la figure 2 est une vue détaillée de l'unité de métathèse de l'installation de la figure 1 ;
- la figure 3 est une vue d'un détail d'une deuxième installation de production destinée à la mise en œuvre d'un procédé selon l'invention.

Dans tout ce qui suit, une même référence désigne un fluide circulant dans une conduite et la conduite qui transporte ce fluide. Par ailleurs, sauf indication contraire, les pourcentages sont des pourcentages molaires et les pressions s'entendent en bars relatifs.

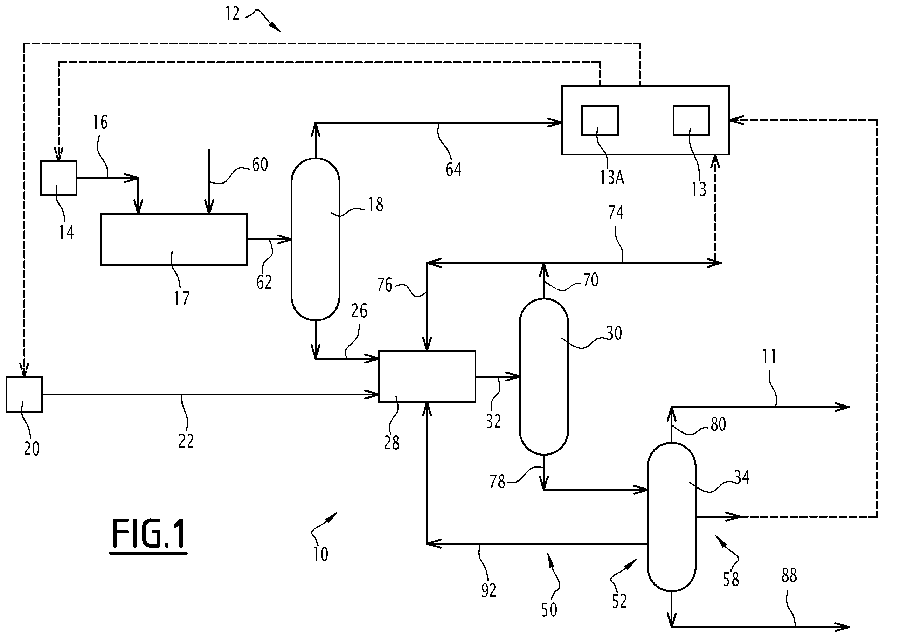

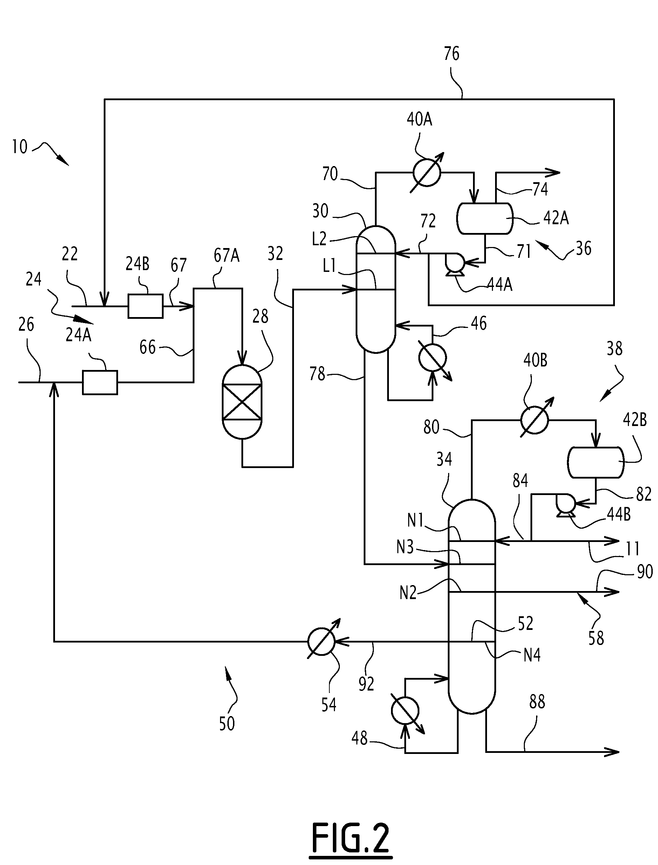

Une première installation 10 de production d'un flux 11 de propylène est illustrée sur les figures 1 et 2. L'installation 10 est intégrée au sein d'une unité 12 de production de dérivés d'hydrocarbures, notamment au sein d'une unité de craquage et de séparation d'hydrocarbures, illustrée partiellement sur la figure 1.

L'unité de production de dérivés d'hydrocarbures 12 comporte au moins un vapocraqueur 13 ou/et une unité 13 de raffinage de type craqueur catalytique ou autres, et des colonnes de distillation 13A, propres à séparer successivement des coupes d'hydrocarbures.

En particulier, l'unité de production de dérivés d'hydrocarbures 12 comporte un équipement 14 de production d'une coupe brute 16 d'hydrocarbures en C4, un équipement 17 d'hydrogénation sélective du butadiène contenu dans la coupe brute 16, assurant également l'hydroisomérisation du 1-butène en 2-butène de la coupe 16, et une colonne 18 de séparation de l'isobutène.

Les équipements 17 et 18 peuvent respectivement être remplacés par des unités d'extraction de butadiène suivie d'une isomérisation 1-butène vers 2-butène (bloc 17) et d'une unité de méthyl tert-butyl éther MTBE (bloc 18).

L'unité de production de dérivés d'hydrocarbures 12 comporte en outre un équipement 20 de production d'une coupe 22 riche en éthylène.

En référence à la figure 2, l'installation 10 comporte au moins un équipement 24 de purification de la coupe riche en éthylène 22 et de purification d'une coupe d'alimentation 26 riche en hydrocarbures oléfiniques en C4 issue de la colonne de séparation 18. L'installation 10 comporte en outre un réacteur de métathèse 28, une première colonne de séparation (dénommée ci-après « Dééthylèniseur 30 »), pour la séparation d'un produit de métathèse 32 produit dans le réacteur 28 et une deuxième colonne de séparation (dénommée ci-après « Dépropylèniseur 34 ») pour la séparation d'un effluent du Dééthylèniseur 30.

Le Dééthylèniseur 30 et le Dépropylèniseur 34 sont chacun munis d'un système de condensation et de reflux respectivement 36 et 38 comprenant chacun un condenseur respectivement 40A, 40B, un séparateur respectivement 42A, 42B et une pompe de reflux respectivement 44A, 44B.

Le Dééthylèniseur 30 et le Dépropylèniseur 34 sont aussi chacun munis d'un rebouilleur de fond respectivement 46 et 48.

L'installation 10 comporte en outre un circuit de recyclage 50 d'un flux riche en hydrocarbures en C4 comportant un ensemble de prélèvement 52 dans le Dépropylèniseur 34, et un échangeur thermique de refroidissement 54 du flux riche en hydrocarbures en C4.

L'installation 10 comporte en outre dans cet exemple un ensemble de soutirage latéral 58, dans le Dépropyleniseur 34, d'un flux riche en hydrocarbures paraffiniques en C4 et/ou riche en isobutène. L'ensemble de soutirage latéral 58 comporte au moins une purge latérale dans le Dépropyleniseur 34.

Un procédé de production de propylène dans l'installation 10 va maintenant être décrit.

Initialement, la coupe brute 16 d'hydrocarbures en C4 est produite à partir du vapocraqueur 13 et/ou d'une unité de raffinage 13 (craqueur catalytique ou autres), et d'étapes de séparation dans l'équipement 14.

Dans le cas où cette coupe 16 est produite uniquement par un vapocraqueur, et avantageusement lorsque ce dernier est opéré à une sévérité moyenne (par exemple P/E=0,61), la coupe C4 comporte des traces de composés C4 acétyléniques (par exemple moins de 5 % molaire), entre 40% molaire et 50% molaire de butadiène (notamment environ 45% molaire), entre 15% molaire et 25% molaire de 1-butène (notamment environ 19% molaire), entre 5% molaire et 15% molaire de 2-butène (notamment environ 10% molaire), entre 15% molaire et 25% molaire d'isobutène (notamment environ 20% molaire) et entre 2% molaire et 10% molaire de composés C4 paraffiniques (notamment environ 6% molaire de n-butane et isobutane).

Les composés pour une coupe issue d'une raffinerie (FCC ou autres) est similaire à l'exception du pourcentage des composés paraffiniques qui est supérieur, par exemple supérieur à 11 % molaire.

Typiquement, la coupe 16 est d'abord introduite dans l'équipement 17 pour éliminer le butadiène par hydrogénation sélective assurant également l'hydroisomérisation du 1-butène en 2-butène. Ainsi, la coupe 16 est mise en contact avec un flux d'hydrogène 60 et un flux hydrogéné 62 d'hydrocarbures en C4 contenant du 1-butène, du 2-butène, de l'isobutène et des C4 paraffiniques est produit. Alternativement, la coupe 62 est produite par extraction du butadiène.

Le flux hydrogéné 62, exempt de butadiène, peut être ensuite traité de plusieurs manières pour produire un flux appauvri en isobutène, grâce par exemple à la colonne de séparation de l'isobutène 18.

Dans ce cas, la colonne 18 produit un flux de tête 64 qui contient l'isobutène séparé, l'isobutane du flux hydrogéné 62 et une partie du 1-butène, puisque le 1-butène présente un point d'ébullition très proche de l'isobutène (moins de 1°C d'écart à pression atmosphérique entre le point d'ébullition de l'isobutène et le point d'ébullition du 1-butène).

La colonne 18 produit en fond un flux constituant la coupe d'alimentation 26.

La coupe d'alimentation 26 contient principalement (par exemple plus de 60% molaire) des normal oléfines, des normal paraffines et, en faible quantité (par exemple moins de 20% molaire) des iso-oléfines et des iso-paraffines.

Alternativement, l'isobutène peut également être enlevé par différentes réactions qui incluent la réaction avec le méthanol pour former le MTBE, la réaction avec l'eau pour former l'alcool tertio-butylique (TBA) ou la réaction avec lui-même pour former un flux C8.

La coupe d'alimentation 26, dans le cas d'une production venant d'un flux C4 de vapocraqueur (opéré avantageusement à un P/E typique de 0,61) traité par hydrogénation puis fractionnement pour enlever l'isobutène, comporte beaucoup de 2-butène (par exemple de l'ordre de 80% molaire ou plus). En variante, selon les différents traitements explicités ci-dessus et/ou l'origine de la coupe C4, la composition en 2-butène est plus réduite.

Dans d'autres variantes, la coupe C4 est mélangée (voire remplacée) par une coupe d'hydrocarbures en C5.

La coupe d'alimentation 26 est riche en hydrocarbures oléfiniques en C4 ou/et en C5, la teneur en hydrocarbures oléfiniques en C4 ou/et en C5 de la coupe d'alimentation 26 étant généralement supérieure à 65% molaire.

La description suivante porte sur une coupe d'alimentation 26 constituée d'hydrocarbures en C4, mais les mêmes développements peuvent être extrapolés à une coupe d'alimentation 26 constituée d'un mélange d'hydrocarbures en C4 et C5, voire à une coupe d'alimentation 26 constituée d'hydrocarbures en C5 uniquement.

En parallèle, la coupe riche en éthylène 22 est produite par exemple à partir du vapocraqueur 13 et d'étapes de séparation dans l'équipement 20.

La coupe riche en éthylène 22, lorsqu'elle est produite à partir d'un vapocraqueur possède typiquement une teneur molaire en éthylène supérieure à 99,9%. Cependant, le procédé ne nécessite pas une pureté si forte et de l'éthylène ayant un grade chimique (teneurs plus faibles i.e. 99% molaire ou moins) est également acceptable.

Ensuite, la coupe d'alimentation 26 et la coupe riche en éthylène 22 sont traitées dans l'équipement 24 afin d'assurer la captation des poisons du catalyseur de métathèse tels que l'eau, le soufre, les alcools, le $CO_2$, les composés azotés, les métaux lourds ....

Selon la provenance des charges et leurs conditions de batterie limite, les équipements sont des lits de gardes 24A, 24B dédiés ou combinés. Usuellement, ils traitent aussi le flux de recyclage d'éthylène 76 et le flux de recyclage 92 riche en hydrocarbures en C4 qui seront décrits plus bas.

Dans l'exemple représenté sur la figure 2, les lits de garde 24A, 24B sont dédiés. La coupe d'alimentation 26 (et le flux de recyclage associé 92) est traitée sur un lit 24A en phase liquide pour former une coupe d'alimentation traitée 66 et la coupe riche en éthylène 22 (et le flux de recyclage associé 76) est traitée sur un lit 24B en phase vapeur pour former une coupe riche en éthylène traitée 67.

La coupe d'alimentation traitée 66 est ensuite mélangée avec la coupe riche en éthylène traitée 67. Le mélange forme une coupe 67A d'alimentation du réacteur 28 qui est vaporisée, chauffée et introduite dans le réacteur de métathèse 28.

En variante (non représentée), la coupe 26 et la coupe 22 (ainsi que les flux respectifs 92 et 76) sont mélangées et introduites dans un même lit de garde 24A opérant en phase liquide, pour former la coupe 67A d'alimentation du réacteur 28. La coupe 67A est envoyée dans le réacteur de métathèse 28 après vaporisation et réchauffage.

D'une manière connue, une réaction de métathèse entre le 2-butène et l'éthylène se produit dans le réacteur de métathèse 28, grâce à la présence d'un catalyseur. Ceci provoque l'intervertissement de fragments entre des molécules de 2-butène et d'éthylène pour former des molécules de propylène à titre de réaction principale. Une réaction de métathèse peut théoriquement intervenir entre tout composé oléfinique, puisqu'il s'agit de l'inversion des groupements alkyles autour des doubles liaisons. Beaucoup de réactions secondaires se produisent donc dans le réacteur. Toutes ces réactions sont régies par les lois d'équilibre.

Afin de favoriser l'équilibre vers la réaction principale, le débit de la coupe riche en éthylène 22 est optimisé de sorte à travailler en excès d'éthylène. Ainsi le ratio des débits molaire d'éthylène et de butène (1-butène et 2-butène) dans la coupe 67A d'alimentation du réacteur est avantageusement compris entre 1,3 et 3.

La réaction de métathèse se produit à une température comprise généralement entre 250°C et 380°C, et à une pression comprise entre 20 bars et 35 bars.

Le catalyseur utilisé comprend généralement une combinaison d'un catalyseur dédié à la métathèse (un métal de transition éventuellement combiné à un oxyde, par exemple l'oxyde de tungstène supporté sur de la silice ou l'oxyde de rhénium supporté sur de l'alumine) et d'un catalyseur dédié à l'isomérisation (un oxyde de métal du groupe II du tableau périodique des éléments, par exemple l'oxyde de magnésium ou l'oxyde de calcium).

Un produit de métathèse 32 est formé à la sortie du réacteur de métathèse 28.

Le produit de métathèse 32 comporte de l'éthylène, du propylène, des hydrocarbures oléfiniques en C4 dont le 1-butène et le 2-butène n'ayant pas réagi, des composés oléfiniques plus lourds et des hydrocarbures paraffiniques.

Le produit de métathèse 32 est ensuite introduit dans le Dééthylèniseur 30 à un premier niveau intermédiaire d'alimentation L1.

La pression régnant dans le Dééthylèniseur 30 est usuellement comprise entre 18 bars et 30 bars. Le Dééthylèniseur 30 est une colonne cryogénique. Le condenseur 40A utilise typiquement des hydrocarbures C3 comme fluide réfrigérant.

Un flux de tête 70 est produit en tête du Dééthylèniseur 30. Le flux de tête 70 contient typiquement une teneur en éthylène supérieur à 95% molaire. Le flux de tête 70 est au moins partiellement condensé dans le condenseur 40A, puis est amené dans le séparateur 42A.

Dans le séparateur 42A, le flux de tête 70 partiellement condensé est séparé en un flux liquide 71 et en une purge vapeur 74.

Le flux liquide 71 récupéré dans le ballon 42 est typiquement pompé et séparé en un reflux 72 et un flux de recyclage d'éthylène 76. La purge vapeur 74 vise à déconcentrer le flux de recyclage d'éthylène 76 des composés légers qui peuvent s'accumuler (méthane, éthane, ...).

L'éthylène récupéré dans le flux de recyclage 76 correspond usuellement à plus de 95% de l'éthylène contenu dans le produit de métathèse 32.

Le reflux 72 est introduit à un niveau L2 du Dééthylèniseur 30, le niveau L2 étant situé au-dessus du niveau d'alimentation L1.

Le courant de recyclage d'éthylène 76 est recyclé vers le réacteur de métathèse 28. Dans l'exemple illustré sur la figure 2, le flux 76 est mélangé à la coupe riche en éthylène 22, en amont du de l'ensemble de traitement 24.

Un flux 78 d'alimentation du Dépropylèniseur 34, est produit en fond du Dééthylèniseur 30.

Ce flux d'alimentation 78 contient du propylène, typiquement environ 99 % du propylène contenu dans le produit de métathèse 32, ainsi que les hydrocarbures en C4 n'ayant pas été convertis dans le réacteur de métathèse 28 et des hydrocarbures en C5+ plus lourds issus des réactions de métathèse secondaires et/ou déjà présents dans la coupe d'alimentation 26.

La teneur en éthylène est presque nulle (par exemple inférieure à 20 ppm en volume en volume) puisqu'elle doit être compatible avec la spécification en éthylène du flux de propylène 11 qui est typiquement très faible (moins de 40 ppm en volume, notamment 30 ppm en volume environ).

Le flux d'alimentation 78 est introduit dans le Dépropylèniseur 34 à un niveau d'alimentation N3.

La pression régnant dans le Dépropylèniseur 34 est usuellement comprise entre 15 bars et 25 bars.

Le Dépropylèniseur 34 produit en tête un flux de tête 80 qui est du propylène à très haute pureté. Avantageusement, le flux de tête 80 présente une spécification typique du propylène grade polymère, supérieure à 99,6% en volume, voire supérieure à 99,9% en volume.

Le flux de tête 80 est au moins partiellement condensé, usuellement en utilisant l'eau comme fluide froid, dans le condenseur 40B, puis est introduit dans le séparateur 42B. Une fraction liquide 82 est prélevée en fond du séparateur 42B, puis est pompée dans la pompe 44B, avant d'être séparée en un reflux 84, et en le flux 11 de propylène. Le reflux 84 est réintroduit dans le Dépropylèniseur 34 à un niveau N1 situé au-dessus du niveau N3.

Comme indiqué plus haut, le flux de propylène 11 présente une « qualité polymère », avec une teneur molaire en propylène généralement supérieure à 99,6% en volume, voire supérieure à 99,9% en volume, une teneur en éthylène généralement inférieure à 50 ppm en volume, typiquement de l'ordre de 30 ppm en volume et une teneur en hydrocarbures en C4 généralement inférieure à 40 ppm en volume, typiquement de l'ordre de 20 ppm en volume.

Le propylène récupéré dans le flux 11 correspond usuellement à plus de 99,5% du propylène contenu dans le flux d'alimentation 78.

Un soutirage d'un flux de recyclage 92 riche en hydrocarbures en C4, contenant la majorité (avantageusement plus de 70%) des composés n-butènes du produit de métathèse 32 n'ayant pas été convertis dans le réacteur de métathèse 28, est prélevé du Dépropylèniseur 34 par l'intermédiaire de l'ensemble de prélèvement 52, à un niveau N4 situé sous le niveau d'alimentation N3.

Usuellement, ce flux de recyclage 92 contient également une partie des hydrocarbures C5 produits dans le réacteur de métathèse 28 et/ou présents dans la coupe d'alimentation 26.

Le flux de recyclage 92 est recyclé vers le réacteur de métathèse 28. Ainsi, il est refroidi dans l'échangeur 54 avant d'être réintroduit dans la coupe 26 d'alimentation en amont de l'équipement de purification 24.

Avantageusement, la quantité de C5 oléfiniques dans le flux de recyclage 92 est optimisée de sorte à limiter, voire bloquer certaines réactions secondaires dans le réacteur de métathèse 28.

Un flux de fond 88 est prélevé au fond du Dépropylèniseur 34. Le flux de fond 88 purge les hydrocarbures en C6 et plus lourds, ainsi que les hydrocarbures en C4 et C5 ne pouvant pas réagir par métathèse.

Le taux de purge est typiquement fixé pour contenir suffisamment de C4 paraffiniques afin d'éviter leur accumulation dans le flux de recyclage 92. En variante, le Dépropylèniseur 34 est suivi d'une autre colonne de distillation (non représentée) recevant le flux de fond 88 pour séparer les composés C4 et C5+.

Selon l'invention, une purge 90 riche en hydrocarbures paraffiniques C4 et/ou riche en isobutène est soutirée du Dépropylèniseur 34 par l'intermédiaire de l'ensemble de soutirage latéral 58, à un niveau de soutirage N2 situé sous le niveau d'alimentation N3, et situé au-dessus du niveau N4 de soutirage du flux de recyclage 92.

La purge 90 contient des composants légers non réactifs tels que les hydrocarbures paraffiniques en C4, en particulier l'isobutane et le n-butane. Il contient également quelques oléfiniques légers non attractifs pour le procédé, en particulier l'isobutène.

La quantité d'hydrocarbures paraffiniques en C4 et/ou d'isobutène dans la purge 90 est généralement supérieure à 50% molaire, notamment supérieure à 60 % molaire.

Les points d'ébullition des hydrocarbures en C4 étant très proches, la purge 90 contient également du 1-butène et du 2-butène. La position de ce soutirage dans le Dépropylèniseur 34 et le débit extrait hors du Dépropylèniseur 34 sont optimisés pour minimiser l'entraînement de ces deux derniers composés.

Le rapport de débit molaire entre la purge 90 et le flux d'alimentation 78 est donc réglé en fonction de la quantité de n-butane, d'isobutane et d'isobutène présents dans le courant d'alimentation 26. Une purge de l'ordre de 90% des hydrocarbures paraffiniques C4 présents dans la coupe d'alimentation 26, est effectuée grâce à la mise en place de la purge 90.

La purge 90 est avantageusement renvoyée dans le vapocraqueur 13 et/ou dans le craqueur catalytique 13.

Ainsi, les hydrocarbures paraffiniques présents dans la purge 90 sont utilisés pour produire des oléfines supplémentaires qui sont séparées en amont de l'installation 10.

Le rendement ultime du procédé de production de propylène par la voie métathèse est généralement défini par :

$$\text{Rendement Ultime} = \frac{\text{Propylène}_{produit}/2}{(1\text{-Butène}+2\text{-Butène})_{\text{Charge Fraiche (coupe 26)}}} \text{ en mol}$$

La conversion du réacteur de métathèse 28 est définie par :

$$\text{Conversion} = \frac{(1\text{-Butène}+2\text{-Butène})_{\text{entrée reacteur}} - (1\text{-Butène}+2\text{-Butène})_{\text{sortie récateur}}}{(1\text{-Butène}+2\text{-Butène})_{\text{entrée réacteur}}}$$

La conversion théorique maximum est très dépendante de la charge puisqu'elle est dictée par l'équilibre de toutes les réactions de métathèse en présence. Dans tous les cas les valeurs attendues sont de l'ordre de 70% maximum, voire moins si la coupe 26 est riche en 1-butène. Il est donc indispensable pour obtenir des rendements ultimes attractifs (par exemple de l'ordre 80% à 90%) de recycler vers le réacteur de métathèse 28 les 1-butène et 2-butène n'ayant pas réagi. Ainsi, à débit du flux de recyclage 92 riche en hydrocarbures en C4 constant :

- plus ce flux 92 sera riche en 1-butène et 2-butène, meilleur sera le rendement ultime ; à l'inverse
- plus ce flux 92 contiendra d'inertes vis-à-vis de la réaction de métathèse (typiquement les paraffines) et/ou de composés susceptibles de limiter la réaction principale du 2-butène vers le propylène (typiquement l'isobutène), moins bon sera le rendement ultime.

Il est donc également induit qu'à rendement ultime constant :

- plus le flux de recyclage 92 sera riche en 1-butène et 2-butène, plus le débit nécessaire sera petit ; à l'inverse
- plus le flux de recyclage 92 contiendra d'inertes vis-à-vis de la réaction de métathèse (typiquement les paraffines) et/ou de composés susceptibles de limiter la réaction principale du 2-butène vers le propylène (typiquement l'isobutène), plus le débit nécessaire sera important.

Selon l'invention, le soutirage d'une purge 90 riche en hydrocarbures paraffiniques en C4 et/ou riche en isobutène réduit le débit du flux de recyclage 92. Ainsi, les dimensions des équipements (et lignes) utilisés pour le recyclage sont diminués de manière significative. De même le débit total transitant dans l'équipement de purification 24 (par exemple les lits de garde 24A, 24B), dans le réacteur de métathèse 28, dans la Dééthylèniseur 30 et dans le Dépropylèniseur 34 sont réduits.

En outre, il n'est pas nécessaire de limiter fortement la teneur en isobutène dans la coupe d'alimentation 26 du réacteur de métathèse 28, puisque l'isobutène est éliminé grâce à la purge 90. Ceci entraîne des économies d'énergie et une réduction de la taille des équipements de pré-traitement, en particulier de la colonne 18 de séparation des isobutènes.

Enfin, le procédé selon l'invention offre une possibilité de dégoulottage de l'installation 10. En effet, comme on l'a vu plus haut, l'ajout d'une purge 90 riche en hydrocarbures paraffiniques C4 et/ou riche en isobutène donne l'opportunité d'opérer l'installation 10 avec un flux 92 de recyclage d'hydrocarbures en C4 limité, et/ou d'améliorer le rendement ultime à même débit de recyclage 92. Cet avantage accroit la production de propylène, en augmentant le débit de la coupe d'alimentation 26 sans avoir à modifier les équipements existants de manière significative.

Le flux de recyclage 92 est soutiré sous forme liquide. En variante, le flux de recyclage 92 riche en hydrocarbures en C4 est avantageusement soutiré en phase gaz. Ainsi, comparativement à un soutirage liquide, la quantité en composants lourds dans le flux de recyclage 92 est fortement diminuée, en particulier celle des hydrocarbures en C6+.

À titre d'illustration, les récupérations dans le Dépropylèniseur 34 des différents composés présents dans le flux de recyclage 92 obtenues à partir d'une coupe d'alimentation 26 telle que décrite dans la Tableau 1 ci-après sont illustrées dans le Tableau 2 suivant. La définition de la récupération R du composé X dans le Dépropylèniseur 34 s'entend selon :

$$R = \frac{(X)_{\text{Recyclage (flux 92)}}}{(X)_{\text{entrée 34 (flux 78)}}} \times 100$$

(X) = débit du composé X

Le Tableau 2 compare une installation de l'état de la technique, dépourvue de purge 90 d'hydrocarbures paraffiniques C4, avec une installation 10 selon l'invention. Deux hypothèses sont présentées pour le soutirage de la purge 90 selon l'invention (soutirage liquide ou soutirage gaz).

La variante dans laquelle le flux de recyclage 92 est gazeux est également présentée dans le Tableau 2.

La coupe d'alimentation 26 présente la composition suivante :

**Tableau 1**

| Propylène | 0,6 |
|---|---|
| Isobutène | 2,1 |
| 1-butène | 5,3 |
| 2-butène | 59,6 |
| n-butane | 26,0 |
| Isobutane | 5,4 |
| Pentènes | 1,0 |

Les données des différentes options du Tableau 2 considèrent toutes un même débit massique du flux de recyclage 92 des hydrocarbures en C4 (typiquement le débit massique du flux de recyclage 92 et considéré identique au débit massique de la coupe d'alimentation 26) et un même excès d'éthylène dans le réacteur de métathèse 28.

**Tableau 2**

| | Récupération dans le Dépropylèniseur 34 selon l'état de la technique (sans purge 90) | Récupération dans le Dépropylèniseur 34 selon l'invention (avec purge 90) | | |
|---|---|---|---|---|
| | | Purge 90 liquide | Purge 90 Gaz | Purge 90 Gaz + Flux de recyclage 92 soutiré gazeux |
| Isobutane | 79 % | 74 % | 71% | 53% |
| Isobutène | 76 % | 74 % | 72 % | 61 % |
| 1-butène | 75 % | 73 % | 72 % | 63 % |
| n-butane | 66 % | 69 % | 70 % | 72 % |
| 2-butène | 65 % | 68 % | 69 % | 73 % |
| Pentènes | 34 % | 36 % | 36 % | 40 % |
| C6+ | 26 % | 25 % | 25 % | 9% |

Grâce à l'implémentation de la purge 90, la quantité de 2-butène récupérée dans le flux de recyclage 92 est significativement plus importante, permettant une meilleure réutilisation de ce composé et donc un meilleur rendement ultime de propylène.

A l'inverse, la quantité de composés paraffiniques légers inertes vis-à-vis de la réaction de métathèse (en particulier, isobutane) et de composés légers susceptibles de limiter la réaction principale du 2-butène vers le propylène (en particulier isobutène) est significativement réduite, évitant le recyclage inutile de ces composés vers le réacteur.

Dans l'exemple présenté, les gains sont encore plus attractifs lorsque la purge 90 est soutirée sous forme gaz plutôt que liquide et lorsque le flux 92 de recyclage d'hydrocarbures en C4 est lui-aussi soutiré sous forme gaz plutôt que liquide.

En outre, la récupération des hydrocarbures C6+ dans le Dépropylèniseur passe d'une valeur typique de l'ordre de 25% lorsque le soutirage du flux de recyclage 92 est liquide à moins de 10% lorsque celui-ci est gaz. Cette forte diminution de la récupération des hydrocarbures C6+ lorsque le flux de recyclage 92 passe de liquide à vapeur est indépendante de la présence de la purge 90 riche en hydrocarbures paraffiniques en C4 et/ou riche en isobutène.

Lorsque le flux de recyclage 92 est soutiré en phase vapeur, les composés lourds sources d'encrassement et de cokage prématuré du catalyseur de métathèse ne sont presque plus recyclés vers le réacteur 28, ce qui allonge la durée de cycle du catalyseur. Comme les régénérations successives à très haute température diminuent l'activité du catalyseur, le catalyseur doit être remplacé au bout de quelques cycles. Un allongement de la durée de cycle allonge donc la durée de vie des catalyseurs.

Les gains attendus à l'implémentation de la purge 90 riche en hydrocarbures paraffiniques en C4 et/ou riche en isobutène dépendent de la composition de la coupe d'alimentation 26. Dans l'exemple présenté ci-dessus (coupe d'alimentation 26 selon le Tableau 1) :

- à même débit du flux de recyclage 92 de la coupe riche en hydrocarbures en C4, le rendement ultime est augmenté de 1,5%
- à même production de propylène, le débit du flux de recyclage 92 de la coupe riche en hydrocarbures en C4 est diminué de 15% environ, ce qui diminue la taille des équipements de purification 24, du réacteur de métathèse 28, du Dééthylèniseur 30 et du Dépropylèniseur 34 de l'ordre de 6%.

Le choix entre une purge 90 sous forme gaz ou liquide est un compromis entre une augmentation de la production et les dépenses d'investissement en capital (CAPEX) puisqu'il conviendra de prendre également en compte la destination finale de la purge 90.

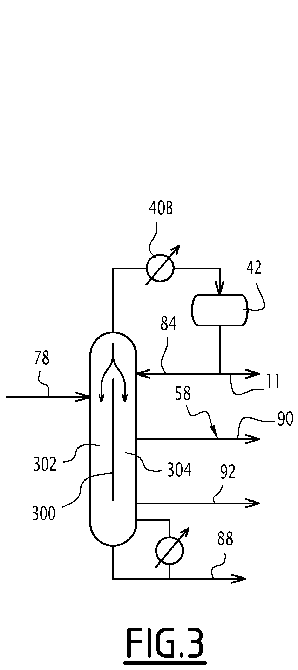

Dans une variante illustrée sur la figure 3, l'installation 10 selon l'invention comporte toujours une purge 90 d'hydrocarbures paraffiniques en C4 et/ou riche en isobutène. Le flux de recyclage 92 riche en hydrocarbures en C4 est prélevé sous forme liquide depuis le Dépropylèniseur 34.

Le Dépropylèniseur 34 comporte alors une colonne de distillation arrangée selon le concept des flux divisés avec paroi de séparation interne 300 verticale, visible sur la figure 3. La paroi de séparation 300 définit dans la colonne de distillation, une première région 302 située d'un côté de la paroi de séparation 300 en regard de l'entrée d'alimentation du flux d'alimentation 78 (à gauche sur la figure 3) et une région opposée 304 située de l'autre côté de la paroi de séparation 300 par rapport à l'entrée d'alimentation du flux d'alimentation 78 (à droite sur la figure 3).

Ainsi, les composés les plus lourds (notamment les hydrocarbures en C6+) du flux d'alimentation 78, transitent directement dans le fond de la colonne de distillation, via la première région 302 (à gauche sur la figure 3) et sont évacués dans le flux de fond 88.

Les composés légers distillent dans la partie haute du même côté de la paroi de séparation 300. Le propylène est extrait en tête dans le flux 11 et les hydrocarbures en C4-C5 poursuivent leur séparation dans la deuxième région 304 définie par la paroi de séparation 300 (à droite sur la figure 3).

La purge 90 riche en hydrocarbures paraffiniques C4 et/ou riche en isobutène est soutirée (sous forme gaz ou liquide) dans la deuxième région 304 définie par la paroi de séparation 300 et le flux de recyclage 92 riche en hydrocarbures en C4 est soutiré en dessous.

Le flux 92 est avantageusement soutiré en phase liquide, tout en assurant une faible récupération des composés lourds, puisque ces derniers ne sont pas présents à cet endroit du Dépropyléniseur 34. L'emploi de la colonne à flux divisés comme Dépropylèniseur 34 allonge les temps de cycle du réacteur de métathèse 28, suivant les mêmes principes que ceux exposés plus haut pour le Dépropylèniseur 34 de la figure 2 avec soutirage du flux de recyclage 92 sous forme gazeuse.

Dans une variante, le procédé est mis en œuvre avec un prélèvement latéral du flux de recyclage 92 riche en hydrocarbures en C4 ou/et en C5 effectué en phase gaz, mais sans purge 90 d'hydrocarbures paraffiniques en C4 et/ou riche en isobutène.

**Revendications**

1. Procédé de production d'un flux de propylène (11), comportant les étapes suivantes :

   - introduction d'une coupe d'alimentation (26) riche en hydrocarbures en C4 ou/et en C5, et d'au moins une coupe (22) riche en éthylène dans un réacteur de métathèse (28) ;
   - récupération d'un produit de métathèse (32) à la sortie du réacteur de métathèse (28) ;
   - introduction du produit de métathèse (32) dans un Dééthylèniseur (30)
   - production en tête du Dééthylèniseur (30) d'un flux de tête (70) riche en éthylène et en fond du Dééthylèniseur (30) d'un flux (78) d'alimentation;
   - introduction du flux d'alimentation (78) dans un Dépropyleniseur (34) et récupération en fond du Dépropylèniseur (34), d'un flux de fond (88) contenant des hydrocarbures en C4+ ;
   - récupération, à partir d'un flux de tête (80) du Dépropylèniseur (34), du flux de propylène (11) ;
   - prélèvement latéral d'un flux de recyclage (92) riche en hydrocarbures en C4 ou/et en C5 et renvoi du flux de recyclage (92) vers le réacteur de métathèse (28) ;

   **caractérisé par** l'étape suivante :

- soutirage latéral, dans le Dépropylèniseur (34), d'une purge (90) riche en hydrocarbures paraffiniques en C4 et/ou riche en isobutène.

2. Procédé selon la revendication 1, comprenant le recyclage de la purge (90) riche en hydrocarbures paraffiniques en C4 et/ou riche en isobutène vers un four de vapocraquage.

3. Procédé selon la revendication 1 ou 2, dans lequel le flux de tête (80) issu du Dépropylèniseur (34) est au moins partiellement condensé pour former une fraction liquide (82), la fraction liquide (82) étant séparée en un reflux (84) introduit à un niveau N1 du Dépropylèniseur (34) et en le flux de propylène (11), la purge (90) riche en hydrocarbures paraffiniques en C4 et/ou riche en isobutène étant soutirée du Dépropylèniseur (34) latéralement à un niveau N2 situé en dessous du niveau N1.

4. Procédé selon l'une quelconque des revendications précédentes, dans lequel le prélèvement latéral du flux de recyclage (92) est effectué à un niveau N4 du Dépropylèniseur (34) situé au-dessous d'un niveau N2 de soutirage latéral de la purge (90) riche en hydrocarbures paraffiniques en C4 et/ou riche en isobutène.

5. Procédé selon l'une quelconque des revendications précédentes, dans lequel le prélèvement latéral du flux de recyclage (92) est effectué en phase gaz.

6. Procédé selon l'une quelconque des revendications précédentes, dans lequel la purge (90) riche en hydrocarbures paraffiniques en C4 et/ou riche en isobutène est soutirée en phase gaz.

7. Procédé selon l'une quelconque des revendications précédentes, comprenant les étapes suivantes :

   - refroidissement et/ou condensation du flux de recyclage (92),
   - introduction du flux de recyclage (92) refroidi et/ou condensé dans la coupe d'alimentation (26).

8. Procédé selon l'une quelconque des revendications précédentes, dans lequel la coupe d'alimentation (26) est formée à partir d'une coupe C4 de vapocraquage, ou/et d'une coupe C4 de raffinerie avantageusement obtenue par craquage catalytique.

9. Installation (10) de production de propylène (11), comportant :

   - un réacteur de métathèse (28) alimenté par au moins une coupe d'alimentation (26) riche en hydrocarbures en C4 ou/et en C5, et par au moins une coupe (22) riche en éthylène, le réacteur de métathèse (28) produisant un produit de métathèse (32) ;
   - un Dééthylèniseur (30), alimenté par le produit de métathèse (32) issu du réacteur de métathèse (28), le Dééthylèniseur (30) produisant, en tête, un flux de tête (70) riche en éthylène et produisant, en fond, un flux d'alimentation (78) ;
   - un Dépropylèniseur (34) recevant le flux d'alimentation (78) et produisant, en fond, un flux de fond (88) contenant des hydrocarbures en C4+ et, en tête, un flux de tête (80) riche en propylène ;
   - un ensemble de récupération, à partir du flux de tête (80) du Dépropylèniseur (34), d'un flux de propylène (11) ;
   - un ensemble de prélèvement latéral (52) d'un flux de recyclage (92) riche en hydrocarbures en C4 ou/et en C5 et de renvoi du flux de recyclage (92) vers le réacteur de métathèse (28) ;

   **caractérisée par** :

   - un ensemble (58) de soutirage latéral, dans le Dépropylèniseur (34), d'une purge (90) riche en hydrocarbures paraffiniques en C4 et/ou riche en isobutène.

10. Installation (10) selon la revendication 9, comprenant au moins un recyclage de la purge (90) riche en hydrocarbures paraffiniques en C4 et/ou riche en isobutène vers un four de vapocraquage.

11. Installation (10) selon la revendication 9 ou 10, comportant un condenseur (40B) propre à au moins partiellement condenser le flux de tête (80) issu du Dépropylèniseur (34) pour former une fraction liquide (82), et un séparateur (42B) propre à fractionner la fraction liquide (82) en un reflux (84) introduit à un niveau N1 du Dépropylèniseur (34) et en le flux de propylène (11), l'ensemble de soutirage (58) de la purge (90) riche en hydrocarbures paraffiniques en C4 et/ou riche en isobutène étant disposé latéralement à un niveau N2 situé en dessous du niveau N1.

12. Installation (10) selon l'une quelconque des revendications 9 à 11, dans laquelle l'ensemble de prélèvement latéral (52) est propre à prélever un flux de recyclage (92) riche en hydrocarbures en C4 en phase gaz.

13. Installation (10) selon l'une quelconque des revendications 9 à 12, dans laquelle l'ensemble de soutirage (58) est propre à soutirer la purge (90) riche en hydrocarbures paraffiniques en C4 et/ou riche en isobutène en phase gaz.

14. Installation (10) selon l'une quelconque des revendications 9 à 12, comprenant:

   - un ensemble de refroidissement et/ou de condensation (54) du flux de recyclage (92),
   - un ensemble d'introduction du flux de recyclage (92) refroidi et/ou condensé dans la coupe d'alimentation (26).

15. Installation (10) selon l'une quelconque des revendications 9 à 14, dans laquelle le Dépropylèniseur (34) comporte une paroi de séparation interne (300) définissant une première région (302) située d'un côté de la paroi de séparation (300) en regard d'une entrée d'alimentation du flux d'alimentation (78) et une région opposée (304) située de l'autre côté de la paroi de séparation (300) par rapport à l'entrée d'alimentation du flux d'alimentation (78), l'ensemble (58) de soutirage latéral de la purge (90) riche en hydrocarbures paraffiniques en C4 et/ou riche en isobutène débouchant dans la région opposée (304).


**Patentansprüche**

1. Verfahren zum Erzeugen eines Propylenstroms (11), umfassend die folgenden Schritte:

   - Einführen eines an C4- und/oder C5-Kohlenwasserstoffen reichen Zufuhrgemischs (26) und mindestens eines an Ethylen reichen Gemischs (22) in einen Metathesereaktor (28);
   - Gewinnen eines Metatheseerzeugnisses (32) an dem Ausgang des Metathesereaktors (28);
   - Einführen des Metatheseerzeugnisses (32) in einen Deethylenisierer (30)
   - Erzeugen eines ethylenreichen Kopfstroms (70) an einem Kopf des Deethylenisierers (30) und eines Zufuhrstroms (78) an einem Boden des Deethylenisierers (30);
   - Einführen des Zufuhrstroms (78) in einen Entpropylenierer (34) und Gewinnen eines Bodenstroms (88), der C4+-Kohlenwasserstoffe enthält, an dem Boden des Entpropylenierers (34);
   - Gewinnen des Propylenstroms (11) aus einem Kopfstrom (80) des Entpropylenierers (34);
   - seitliche Probenahme eines an C4- und/oder C5-Kohlenwasserstoffen reichen Rückführstroms (92) und Leiten des Rückführstroms (92) zu dem Metathesereaktor (28);

   **gekennzeichnet durch** den folgenden Schritt:

   - seitliches Abziehen einer an C4-Paraffinkohlenwasserstoffen reichen und/oder an Isobuten reichen Spülung (90) in dem Entpropylenierer (34).

2. Verfahren nach Anspruch 1, umfassend das Rückführen der an C4-Paraffinkohlenwasserstoffen reichen und/oder an Isobuten reichen Spülung (90) zu einem Dampfcrackofen.

3. Verfahren nach Anspruch 1 oder 2, wobei der Kopfstrom (80), der aus dem Entpropylenierer (34) stammt, mindestens teilweise kondensiert wird, um eine flüssige Fraktion (82) auszubilden, wobei die flüssige Fraktion (82) in einen Rückfluss (84), der auf einer Ebene N1 des Entpropylenierers (34) eingeführt wird, und in den Propylenstrom (11) aufgeteilt wird, wobei die an C4-Paraffinkohlenwasserstoffen reiche und/oder an Isobuten reiche Spülung (90) seitlich auf einer Ebene N2, die sich unterhalb der Ebene N1 befindet, aus dem Entpropylenierer (34) abgezogen wird.

4. Verfahren nach einem der vorstehenden Ansprüche, wobei die seitliche Probenahme des Rückführstroms (92) auf einer Ebene N4 des Entpropylenierers (34) durchgeführt wird, die sich unterhalb einer Ebene N2 des seitlichen Abziehens der an C4-Paraffinkohlenwasserstoffen reichen und/oder an Isobuten reichen Spülung (90) befindet.

5. Verfahren nach einem der vorstehenden Ansprüche, wobei die seitliche Probenahme des Rückführstroms (92) in der Gasphase durchgeführt wird.

6. Verfahren nach einem der vorstehenden Ansprüche, wobei die an C4-Paraffinkohlenwasserstoffen reiche und/oder an Isobuten reiche Spülung (90) in der Gasphase abgezogen wird.

7. Verfahren nach einem der vorstehenden Ansprüche, umfassend die folgenden Schritte:

   - Kühlen und/oder Kondensieren des Rückführstroms (92),
   - Einführen des gekühlten und/oder kondensierten Rückführstroms (92) in das Zufuhrgemisch (26).

8. Verfahren nach einem der vorstehenden Ansprüche, wobei das Zufuhrgemisch (26) aus einem C4-Dampfcrackgemisch und/oder einem vorteilhafterweise durch katalytisches Cracken erhaltenen C4-Raffineriegemisch ausgebildet wird.

9. Anlage (10) zum Erzeugen von Propylen (11), umfassend:

   - einen Metathesereaktor (28), der durch mindestens ein an C4- und/oder C5-Kohlenwasserstoffen reiches Zufuhrgemisch (26) und durch mindestens ein ethylenreiches Gemisch (22) gespeist wird, wobei der Metathesereaktor (28) ein Metatheseerzeugnis (32) erzeugt;
   - einen Deethylenisierer (30), der durch das Metatheseerzeugnis (32), das aus dem Metathesereaktor (28) stammt, gespeist wird, wobei der Deethylenisierer (30) an dem Kopf einen ethylenreichen Kopfstrom (70) erzeugt und an dem Boden einen Zufuhrstrom (78) erzeugt;
   - einen Entpropylenierer (34), der den Zufuhrstrom (78) aufnimmt und an dem Boden einen Bodenstrom (88), der C4+-Kohlenwasserstoffe enthält, und an dem Kopf einen propylenreichen Kopfstrom (80) erzeugt;
   - eine Anordnung zum Gewinnen eines Propylenstroms (11) aus dem Kopfstrom (80) des Entpropylenierers (34);
   - ein Anordnung (52) für seitliche Probenahme eines an C4- und/oder C5-Kohlenwasserstoffen reichen Rückführstroms (92) und Leiten des Rückführstroms (92) zu dem Metathesereaktor (28);

   **gekennzeichnet durch:**

   - eine Anordnung (58) zum seitlichen Abziehen einer an C4-Paraffinkohlenwasserstoffen reichen und/oder an Isobuten reichen Spülung (90) in dem Entpropylenierer (34).

10. Anlage (10) nach Anspruch 9, umfassend mindestens ein Rückführen der an C4-Paraffinkohlenwasserstoffen reichen und/oder an Isobuten reichen Spülung (90) zu einem Dampfcrackofen.

11. Anlage (10) nach Anspruch 9 oder 10, aufweisend einen Kondensator (40B), der geeignet ist, den Kopfstrom (80), der aus dem Entpropylenierer (34) stammt, mindestens teilweise zu kondensieren, um eine flüssige Fraktion (82) auszubilden, und einen Abscheider (42B), der geeignet ist, die flüssige Fraktion (82) in einen Rückfluss (84), der auf einer Ebene N1 des Entpropylenierers (34) eingeführt wird, und in den Propylenstrom (11) zu fraktionieren, wobei die Anordnung (58) zum Abziehen der an C4-Paraffinkohlenwasserstoffen reichen und/oder an Isobuten reichen Spülung (90) seitlich auf einer Ebene N2 angeordnet ist, die sich unterhalb der Ebene N1 befindet.

12. Anlage (10) nach einem der Ansprüche 9 bis 11, wobei die Anordnung (52) für seitliche Probenahme geeignet ist, an einem an C4-Kohlenwasserstoffen reichen Rückführstrom (92) in der Gasphase eine Probe zu entnehmen.

13. Anlage (10) nach einem der Ansprüche 9 bis 12, wobei die Abziehanordnung (58) geeignet ist, die an C4-Paraffinkohlenwasserstoffen reiche und/oder an Isobuten reiche Spülung (90) in der Gasphase abzuziehen.

14. Anlage (10) nach einem der Ansprüche 9 bis 12, umfassend:

   - eine Anordnung (54) zum Kühlen und/oder Kondensieren des Rückführstroms (92),
   - eine Anordnung zum Einführen des gekühlten und/oder kondensierten Rückführstroms (92) in das Zufuhrgemisch (26).

15. Anlage (10) nach einem der Ansprüche 9 bis 14, wobei der Entpropylenierer (34) eine innere Trennwand (300) aufweist, die einen ersten Bereich (302), der sich auf einer Seite der Trennwand (300) gegenüber einem Zufuhreinlass des Zufuhrstroms (78) befindet, und einen gegenüberliegenden Bereich (304), der sich auf der anderen Seite der Trennwand (300) relativ zu dem Zufuhreinlass des Zufuhrstroms (78) befindet, definiert, wobei die Anordnung (58) zum seitlichen Abziehen der an C4-Paraffinkohlenwasserstoffen reichen und/oder an Isobuten reichen Spülung (90) in den gegenüberliegenden Bereich (304) mündet.

**Claims**

1.  Method for producing a stream (11) of propylene, comprising the following steps:

    - introducing a feed cut (26) rich in C4 and/or C5 hydrocarbons and at least one cut (22) rich in ethylene into a metathesis reactor (28);
    - recovering a metathesis product (32) at the outlet of the metathesis reactor (28);
    - introducing the metathesis product (32) into a Deethylenizer (30)
    - producing an ethylene-rich overhead stream (70) at the top of the Deethylenizer (30) and a feed stream (78) at the bottom of the Deethylenizer (30);
    - introducing the feed stream (78) into a Depropylenizer (34) and recovering, at the bottom of the Depropylenizer (34), a bottom stream (88) containing C4+ hydrocarbons;
    - recovering the propylene stream (11) from an overhead stream (80) of the Depropylenizer (34);
    - lateral removal of a recycle stream (92) rich in C4 and/or C5 hydrocarbons and returning the recycle stream (92) to the metathesis reactor (28);

    **characterized by** the following step:

    - lateral withdrawal, in the Depropylenizer (34), of a purge (90) rich in C4 paraffinic hydrocarbons and/or rich in isobutene.

2.  Method according to claim 1, comprising recycling the purge (90) rich in C4 paraffinic hydrocarbons and/or rich in isobutene to a steam cracking furnace.

3.  Method according to claim 1 or 2, wherein the overhead stream (80) from the Depropylenizer (34) is at least partially condensed to form a liquid fraction (82), the liquid fraction (82) being separated into a reflux (84) introduced at a level N1 of the Depropylenizer (34) and the propylene stream (11), the purge (90) rich in C4 paraffinic hydrocarbons and/or rich in isobutene being withdrawn from the Depropylenizer (34) laterally at a level N2 located below the level N1.

4.  Method according to any one of the preceding claims, wherein the lateral removal of the recycle stream (92) is carried out at a level N4 of the Depropylenizer (34) located below a level N2 for lateral withdrawal of the purge (90) rich in C4 paraffinic hydrocarbons and/or rich in isobutene.

5.  Method according to any one of the preceding claims, wherein the lateral removal of the recycle stream (92) is carried out in the gas phase.

6.  Method according to any one of the preceding claims, wherein the purge (90) rich in C4 paraffinic hydrocarbons and/or rich in isobutene is withdrawn in the gas phase.

7.  Method according to any one of the preceding claims, comprising the following steps:

    - cooling and/or condensation of the recycle stream (92),
    - introducing the cooled and/or condensed recycle stream (92) into the feed cut (26).

8.  Method according to any one of the preceding claims, wherein the feed cut (26) is formed from a C4 steam cracking cut, and/or a C4 refinery cut advantageously obtained by catalytic cracking.

9.  Plant (10) for producing propylene (11), comprising:

    - a metathesis reactor (28) supplied with at least one feed cut (26) rich in C4 and/or C5 hydrocarbons and with at least one cut (22) rich in ethylene, the metathesis reactor (28) producing a metathesis product (32);
    - a Deethylenizer (30), supplied with the metathesis product (32) from the metathesis reactor (28), the Deethylenizer (30) producing, at the top, an ethylene-rich overhead stream (70) and producing, at the bottom, a feed stream (78);
    - a Depropylenizer (34) receiving the feed stream (78) and producing, at the bottom, a bottom stream (88) containing C4+ hydrocarbons and, at the top, a propylene-rich overhead stream (80);
    - a unit for recovering a propylene stream (11) from the overhead stream (80) of the Depropylenizer (34);
    - a unit (52) for laterally removing a recycle stream (92) rich in C4 and/or C5 hydrocarbons and returning the recycle stream (92) to the metathesis reactor (28);

**characterized by**:

- a unit (58) for lateral withdrawal, in the Depropylenizer (34), of a purge (90) rich in C4 paraffinic hydrocarbons and/or rich in isobutene.

10. Plant (10) according to claim 9, comprising at least one recycling of the purge (90) rich in C4 paraffinic hydrocarbons and/or rich in isobutene to a steam cracking furnace.

11. Plant (10) according to claim 9 or 10, comprising a condenser (40B) capable of at least partially condensing the overhead stream (80) from the Depropylenizer (34) to form a liquid fraction (82), and a separator (42B) capable of fractionating the liquid fraction (82) into a reflux (84) introduced at a level N1 of the Depropylenizer (34) and the propylene stream (11), the unit (58) for withdrawing the purge (90) rich in C4 paraffinic hydrocarbons and/or rich in isobutene being arranged laterally at a level N2 located below the level N1.

12. Plant (10) according to any one of claims 9 to 11, wherein the lateral removal unit (52) is capable of removing a recycle stream (92) rich in C4 hydrocarbons in the gas phase.

13. Plant (10) according to any one of claims 9 to 12, wherein the withdrawal unit (58) is capable of withdrawing the purge (90) rich in C4 paraffinic hydrocarbons and/or rich in isobutene in the gas phase.

14. Plant (10) according to any one of claims 9 to 12, comprising:

- a unit (54) for cooling and/or condensation of the recycle stream (92),
- a unit for introducing the cooled and/or condensed recycle stream (92) into the feed cut (26).

15. Plant (10) according to any one of claims 9 to 14, wherein the Depropylenizer (34) comprises an internal partition wall (300) defining a first region (302) located on one side of the partition wall (300) facing a feed inlet for the feed stream (78) and an opposite region (304) located on the other side of the partition wall (300) from the feed inlet for the feed stream (78), the unit (58) for lateral withdrawal of the purge (90) rich in C4 paraffinic hydrocarbons and/or rich in isobutene opening into the opposite region (304).

FIG.1

EP 3 797 096 B1

FIG.2

FIG.3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2017003818 A